# EUROPEAN PATENT APPLICATION

(11) **EP 3 939 703 A2**
(43) Date of publication of application: **19.01.2022**
(21) Application number: 20770843.9
(22) Date of filing: 07.02.2020
(51) Int. Cl.: B01L 7/00

(54) **INDEPENDENTLY TEMPERATURE CONTROLLABLE BLOCK FOR PCR**

(30) Priority: 12.03.2019 KR 20190028389
(71) Applicant: Future-Eng Co., Ltd., Yuseong-gu Daejeon 34015 (KR)
(72) Inventor: LEE, Hyo, Daejeon 34010 (KR)
(74) Representative: Pfenning, Meinig & Partner mbB
(86) International application number: PCT/KR2020/001766
(87) International publication number: WO 2020/184844

(57) **Abstract**

The present invention relates to an independently temperature controllable block for PCR. More specifically, the purpose of the present invention is to provide a temperature circulation apparatus which is for PCR and has a plurality of individual heat chambers, wherein: the upper and lower ends of a plurality of arrayed heat-generating chambers can each be divided into rows and columns and selectively electrified; temperature measurement sensors are provided to match one-to-one with the heat-generating chambers, and thus each of the heat-generating chambers can be independently and accurately controlled; a conventional heat chamber can be replaced merely by coupling a heat-generating chamber, coated with a heat-generating material, between upper and lower end boards having a printed circuit board configuration, thus simplifying the structure and reducing manufacturing costs; a heat-generating chamber having a wide top and narrow bottom shape can be heated directly to transfer the heat directly to a tube body closely attached to the inside; and heating and cooling can be rapidly performed due to the low heat capacity of the heat-generating chamber, and thus a temperature cycling time can be reduced.

## Description

### Technical Field

The present invention relates to a block for PCR capable of independent temperature control and, more specifically, to a temperature cycling device for PCR with multiple single heating chambers, in which an array of a plurality of heating chambers are divided into columns and rows at their upper and lower ends to be selectively electrically conducted, and the heating chambers may be independently and precisely controlled for their temperature, and conventional heating chambers may be replaced simply by coupling heating element-coated heating chambers between an upper board and a lower board, which are shaped as printed circuit boards to thus simplify the structure and save manufacturing costs, and the heating chambers, which have a wide-top-narrow-bottom shape are directly heated to directly conduct heat to tube bodies in tight contact with the inside of the heating chambers, and the heating chambers have low heat capacity to thus allow for rapid heating and cooling to thereby shorten the temperature cycling time.

### Background Art

A conventional thermal cycler which used for gene amplification consists of a hot lid to prevent evaporation of the sample in the tube, a block for mounting a samplecontaining tube or plate, and a heat supply device for temperature cycling of the block.

The heat supply device needs to be capable of heating and cooling, and for this purpose, a thermoelectric element or a heat exchange device has been used. Recently, for a simplified device and easier control, Peltier elements are mostly used to cycle the temperature of the tube-mounted block.

In a thermal cycler, which uses a Peltier element for temperature cycling, one surface of the Peltier element, which contacts the block, has opposite thermal characteristics to those of the opposite surface. In other words, if the surface of the Peltier element, which is attached to the block, is heated up, the opposite surface is cooled down and vice versa. Heating or cooling is determined depending on the direction of the current supplied to the Peltier element and, if the difference in temperature between the two opposite surfaces of the Peltier element does not remain a predetermined temperature or more, the temperature of the target surface does not change. Thus, a heat radiating plate is typically attached to the surface of the Peltier element, opposite to the block, to lead to a temperature difference of the predetermined temperature or more.

Conventional blocks are formed to fit the shape of the tube or plate and thus have a substantially constant shape or size. A plurality of Peltier elements are attached according to the size of the block and are connected in series or parallel, and the amount and direction of current are adjusted to change or maintain the temperature.

In this case, typically four or six Peltier elements are attached to the block. Since the temperature may not differently controlled on one Peltier surface, the same amount of heat is supplied to the area of the block corresponding to the size of the Peltier surface. However, a temperature deviation from the central part occurs around the block due to temperature disturbance due to contact with external air or equipment.

In the thermal cycler for PCR, the main factor that affects gene amplification is the temperature of the block. In the case of using a Peltier element, the central part and the periphery cannot be individually controlled for temperature, and a difference in temperature between the central part and the periphery is inevitable.

The block needs to be constructed in an integrated form to reduce the temperature deviation between wells and to be mechanically attached to the Peltier surface. For this reason, the heat capacity of the block is increased, and as a result, more energy than necessary for temperature cycling of the gene sample is consumed, and more time is required for temperature cycling.

Therefore, it is necessary to develop a temperature cycling device for PCR that may independently control the temperature of each tube containing the gene sample and may quickly perform temperature cycling by reducing heat capacity.

### [Prior Technical Documents]

### [Patent Documents]

(Patent Document 1) KR 10-0603821 published on July 14, 2006.

### Detailed Description of the Present Invention

### Technical Problems

The present invention aims to provide a temperature cycling device for PCR having a plurality of single heating chambers which may reduce the heat capacity of block, independently control the temperature for each heating chamber, and perform precise and rapid temperature cycling.

The present invention also aims to provide a temperature cycling device for PCR, including a plurality of single heating chambers which are divided into columns and rows at their upper and lower ends to be selectively electrically conducted so that the temperature of each of the heating chambers may be independently controlled.

The present invention also aims to provide a temperature cycling device for PCR, including a plurality of single heating chambers in which the conventional heating chambers may be replaced simply by coupling the heating chambers coated with the heating elements between the upper board and the lower board which are shaped as printed circuit boards, thus rendering it possible to simplify the structure and save manufacturing costs.

The present invention also aims to provide a temperature cycling device for PCR having a plurality of single heating chambers in which the heating chambers having a wide-top-narrow-bottom shape are directly heated, and heat is directly transferred to the tube bodies tightly contacting the inside thereof and thus, the thermal conductance time is short, and the heat capacity of the heating chambers is small, so that heating and cooling may be quickly performed. Thus, it is possible to shorten the temperature cycling time.

### Means to Address the Problems

The present invention comprises an upper board including an array of a plurality of upper through holes, upper electrodes formed at the upper holes, and a plurality of upper power lines connected to the upper electrodes, a lower board including an array of a plurality of lower through holes, lower electrodes formed at the lower holes, and a plurality of lower power lines connected to the lower electrodes, a temperature measurement board disposed under the lower board and including a plurality of temperature measurement sensors, a plurality of heating chambers including chamber bodies whose lower surfaces contacting the temperature measurement sensors, heating elements formed on outer circumferential surfaces of the chamber bodies to receive electricity to generate heat, upper rings provided on the chamber bodies to be electrically conducted with the heating elements and contacting the upper electrodes, and lower rings provided on the chamber bodies to be electrically conducted with the heating chambers and contacting the lower electrodes, and a controller selectively conducting electricity to the upper electrodes and the lower electrodes to allow the heating elements of the heating chambers to be selected heated.

According to the present invention, the controller includes power source switches individually provided to the upper power lines to connect the upper power lines to a power source and ground switches individually provided to the lower power lines to connect the lower power lines to a ground.

According to the present invention, the temperature measurement sensors have heating addresses using sequence numbers of the upper power lines and the lower power lines, as serial numbers. The controller receives measurement values from the temperature measurement sensors, matches pulse widths of voltages to be supplied to the heating chambers with the heating addresses using differences between the measurement values of the temperature measurement sensors and a target temperature value and stores the matches, and when an upper power line and a lower power line are connected to the power source and the ground, controls the power source switches and the ground switches to apply power having a pulse width corresponding to a heating address to the upper power line and the lower power line.

The present invention further comprises a bracket including a top opening partially formed in an upper surface to expose the upper holes of the upper board to an outside and a bottom opening formed in a central portion of a lower surface; and a suction motor coupled to a lower end of the bottom opening of the bracket to suck air from an inside of the bracket. The temperature measurement board has a plurality of through holes in a position corresponding to the bottom opening. The bracket has air inlets formed in a pair of directions symmetrical with each other not to contact the temperature measurement board or the upper board, external air introduced through the air inlets.

According to the present invention, the chamber bodies of the heating chambers are formed in a wide-top-narrow bottom shape.

According to the present invention, the upper rings and the lower rings of the heating chambers are formed of a material capable of conductance and soldering. The heating elements are formed by coating the chamber bodies with a heat-generative paste, with the upper rings and the lower rings coupled.

### Effects of the Invention

According to the present invention, the plurality of heating chambers are independently arrayed, and power is applied to the heating elements formed on the outer circumferential surfaces of the heating chambers to heat the tube bodies. Thus, heating may be quickly performed, and each heating chamber may be independently heated, rendering it possible to precisely control temperature.

Further, according to the present invention, the plurality of heating chambers, which are arrayed, are divided into columns and rows at their upper and lower ends and may be selectively connected, so that each of the heating chambers may be independently controlled for its temperature.

Further, according to the present invention, since a different temperature may be set for each heating chamber, it is possible to identify the reactivity for multiple temperature conditions by one test.

Further, according to the present invention, since a different temperature may be set for each heating chamber, it is possible to simultaneously perform gene amplification tests with different temperature conditions.

Further, according to the present invention, since the conventional heating chambers may be replaced simply by coupling the heating chambers coated with the heating elements between the upper board and the lower board which are shaped as printed circuit boards, it is possible to simplify the structure and save manufacturing costs.

Further, according to the present invention, the heating chambers having a wide-top-narrow-bottom shape are directly heated, and heat is directly transferred to the tube bodies tightly contacting the inside thereof. Thus, the thermal conductance time is short, and the heat capacity of the heating chambers is small, so that heating and cooling may be quickly performed. Thus, it is possible to shorten the temperature cycling time.

### Brief Description of the Drawings

FIG. 1 is a perspective view illustrating an inside of an independently temperature controllable block for PCR;
FIG. 2 is an exploded perspective view illustrating an inside of an independently temperature controllable block for PCR;
FIG. 3 is an enlarged view illustrating a main portion of an independently temperature controllable block for PCR;
FIG. 4 illustrates a perspective view and an exploded perspective view of an independently temperature controllable block for PCR;
FIG. 5 is a perspective view illustrating an independently temperature controllable block for PCR;
FIG. 6 is a view illustrating an example of an operation for cooling an independently temperature controllable block for PCR; and
FIG. 7 is a view illustrating an example of an operation for cooling an independently temperature controllable block for PCR.

### Best Mode to Practice the Invention

Hereinafter, embodiments of the present invention are described in detail with reference to the accompanying drawings.

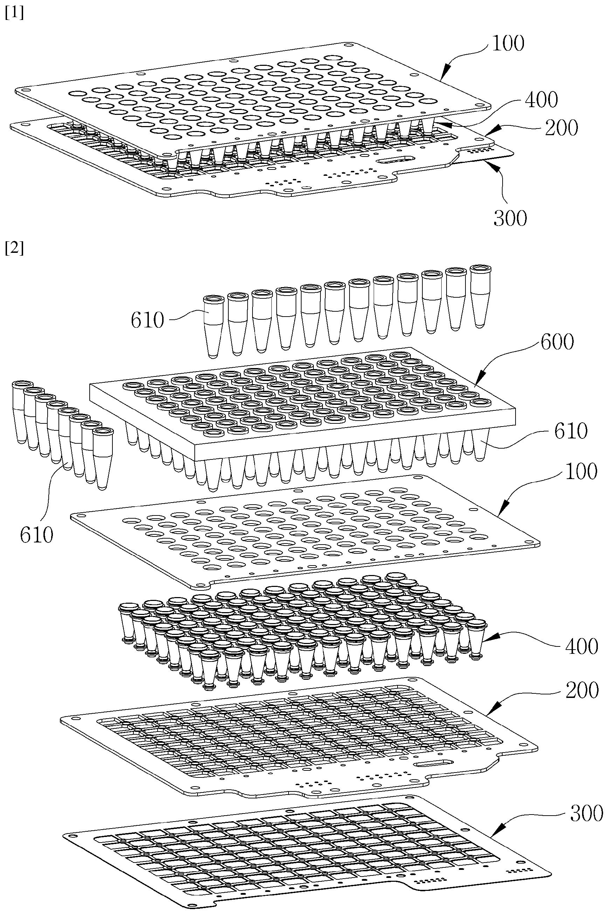

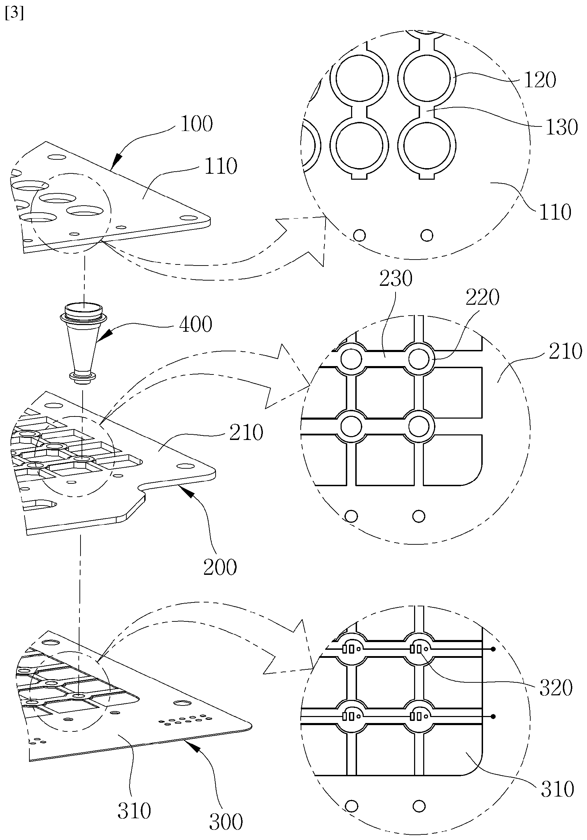

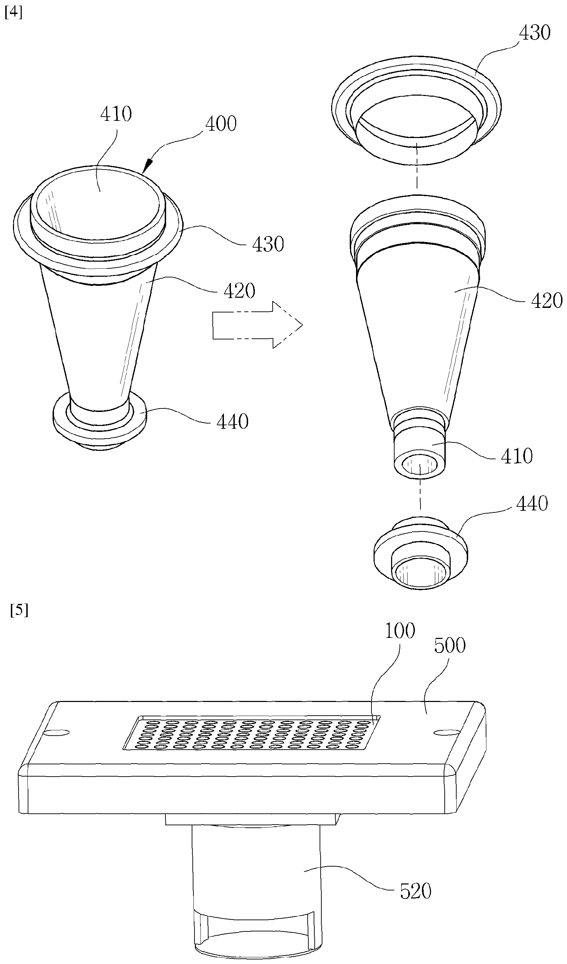

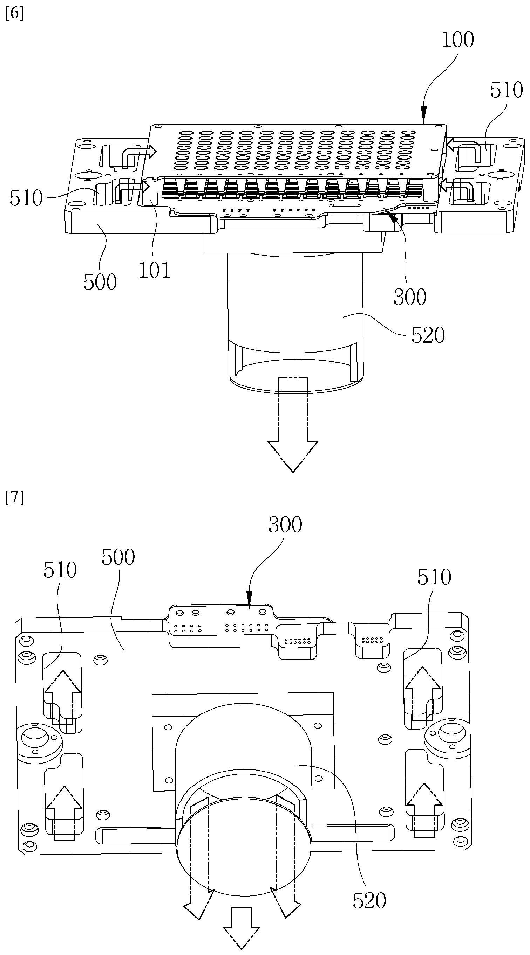

According to the present invention, referring to FIGS. 1 to 7, a block for PCR includes an upper board 100 including an array of a plurality of upper through holes, upper electrodes 120 formed at the upper holes, and a plurality of upper power lines 130 connected to columns of the upper electrodes 120, a lower board 200 including an array of a plurality of lower through holes, lower electrodes 220 formed at the lower holes, and a plurality of lower power lines 230 connected to rows of the lower electrodes 220, a temperature measurement board 300 disposed under the lower board 200 and including an array of a plurality of temperature measurement sensors 320, heating chambers 400, each of which has an upper end, with a top opening, and a lower end inserted into the upper hole of the upper board 100 and the lower hole of the lower board 200, respectively, and including heating elements 420 formed on outer circumferential surfaces thereof to be electrically conducted to the upper electrodes 120 and the lower electrodes 220, a sample plate 600 having a plurality of tube bodies 610 insertable into the heating chambers 400, and a controller selectively connecting the upper power lines 130 and the lower power lines 230 to a power source and a ground, respectively, to allow the heating elements 420 of the heating chambers 400 to generate heat.

The upper board 100 provides power lines to selectively connect the power source to the heating elements 420 of the heating chambers 400 and, to that end, the upper board 100 includes an upper plate 110, the array of upper through holes formed in the upper plate, the upper electrodes 120 individually formed at the upper holes, and the plurality of upper power lines 130 individually connected to the columns of the upper electrodes 120.

The upper plate 110 may be a typical plate-shaped printed circuit board (PCB), and the plurality of upper holes may be formed therethrough.

The upper electrodes 120, along with the upper power lines 130, may be formed on the upper plate 110 by printing. The upper electrode 120 contacts the upper ring 430 of the heating chamber 400 and is electrically conducted, and the upper power line 130 connects an external power source up to the upper electrode 120.

The plurality of upper power lines 130 electrically connect the upper electrodes 120 along the columns thereof. The upper power lines 130 may be formed on the upper plate 110 by plating, and a contact terminal may be formed on one side of the upper plate 110 to connect each upper power line 130 to the outside. The upper power lines 130 may be formed only on the upper surface of the upper plate 110 or only on the lower surface of the upper plate 110, or some of the upper power lines 130 may be formed on the upper surface of the upper plate 110 while others of the upper power lines 130 may be formed on the lower surface of the upper plate 110. Each of the upper power lines 130 is connected to the power source via a power source switch and is assigned a unique number, by the controller, for power source connection control.

The lower board 200 provides power lines to selectively connect the ground to the heating elements 420 of the heating chambers 400 and, to that end, the lower board 200 is disposed under the upper board 100 and includes a lower plate 210, the array of lower through holes formed in the lower plate 210, the lower electrodes 220 individually formed at the lower holes, and the plurality of lower power lines 230 individually connected to the rows of the lower electrodes 220.

The lower plate 210 may be a typical plate-shaped printed circuit board, and the plurality of lower holes may be formed therethrough.

The lower electrodes 220, along with the lower power lines 230, may be formed on the lower plate 210 by printing. The lower electrode 220 contacts the lower ring 440 of the heating chamber 400 and is electrically conducted, and the lower power line 230 connects an external power source up to the lower electrode 220.

The plurality of lower power lines 230 electrically connect the lower electrodes 220 along the rows thereof. The lower power lines 230 may be formed on the lower plate 210 by plating, and a contact terminal may be formed on one side of the lower plate 210 to connect each lower power line 230 to the outside. The lower power lines 230 may be formed only on the upper surface of the lower plate 210 or only on the lower surface of the lower plate 210, or some of the lower power lines 230 may be formed on the upper surface of the lower plate 210 while others of the lower power lines 230 may be formed on the lower surface of the lower plate 210. Each of the lower power lines 230 is connected to the ground via a ground switch and is assigned a unique number, by the controller, for ground connection control.

Although the upper board 100 and the lower board 200 are described so that the upper power lines 130 may be arrayed in columns, and the lower power lines 230 may be arrayed in rows, it is apparent to one of ordinary skill in the art that such a structure in which the rows and the columns of the upper board 100 and the lower board 200 are interchanged is also possible as long as the structure may apply power to the heating chambers 400 in the crossings of the upper power lines 130 and the lower power lines 230 through the upper power lines 130 and the lower power lines 230. For the reasons, the upper power lines 130 may be connected to the ground, and the lower power lines 230 may be connected to the power source. It is apparent to one of ordinary skill in the art that, although the description has been made definitely, not in a selective way, electric heating elements without directivity may be used, with the two polarities changed.

The temperature measurement board 300 measures the heat from the chamber body 410 of the heating chamber 400 and, to that end, includes a temperature measurement plate 310 disposed under the lower board 200 and an array of a plurality of temperature measurement sensors 320 formed on the temperature measurement plate 310.

The temperature measurement plate 310 may be a typical plate-shaped printed circuit board and has the plurality of temperature measurement sensors 320 arrayed thereon. Measurement lines connected with the temperature measurement sensors 320 may be formed, by printing, on the temperature measurement plate 310. The temperature measurement sensor 320 may be a negative temperature coefficientthermal resistor (NTC-thermistor) having a negative (-) temperature coefficient (α), whose resistance reduces as the ambient temperature increases, but is not limited thereto.

The temperature measurement sensors 320 contact the lower ends of the chamber bodies 410 of the heating chambers 400 to measure the temperature of the chamber bodies 410 and, to that end, are provided in the same array as that of the heating chambers 400. To effectively conduct the heat from the chamber body 410 to the temperature measurement sensor 320, a thermal conductive coupling material, e.g., thermal conductive silicone, may be provided and thermally connected between the chamber body 410 and the temperature measurement sensor 320.

The temperature measurement sensor 320 has a heating address that uses, as its serial number, the sequence number of the upper power line 130 and the sequence number of the lower power line 230. The heating address is described below in connection with the controller.

The temperature measurement plate 310 may be perforated in the rest except for where the temperature measurement sensors 320 and the measurement lines are formed. The perforations may provide air passages for sucking in or discharging the air by a suction motor 520 described below.

The heating chamber 400 receives power and generates heat, thereby heating the tube body 610 inserted therein. To that end, the heating chamber 410 includes a chamber body 410, a heating element 420, an upper ring 430, and a lower ring 440. The chamber body 410 has an upper end inserted to the upper hole of the upper board 100 and a lower end inserted to the lower hole of the lower board 200. The upper end of the chamber body 410 has a cylindrical shape with a top opening. The heating element 420 is formed on the outer circumferential surface of the chamber body 410 and receiving electricity to generate heat. The upper ring 430 is provided on the outer circumferential surface of the upper end of the chamber body 410 to be electrically connected with the heating element 420 and contacts the upper electrode 120 when the upper end of the chamber body 410 is inserted to the upper hole. The lower ring 440 is provided on the outer circumferential surface of the lower end of the chamber body 410 to be electrically connected with the heating element 420 and contacts the lower electrode 220 when the lower end of the chamber body 410 is inserted to the lower hole.

The chamber body 410 is coated with insulation, provides a space in which the tube body 610 may be inserted, and transfer the heat generated from the heating element 420 to the tube body 610. To that end, the chamber body 410 is shaped as a wide-top-narrow-bottom, inverted cone, and has a top opening through which the tube body 610 described below may be inserted.

The upper end of the chamber body 410 is inserted to the upper hole of the upper board 100, and the lower end of the chamber body 410 is inserted to the lower hole of the lower board 200, and the chamber body is fixed. In this case, the upper ring coupled to the upper end of the chamber body 410 and the lower ring coupled to the lower end of the chamber body 410 may be interposed between the chamber body 410 and the upper hole or the lower hole, but embodiments are not limited thereto.

The heating element 420 which receives power and generates heat is formed on the outer circumferential surface of the chamber body 410.

When power is applied to the heating element 420, the heating element 420 generates heat and heats the chamber body 410, thus heating the tube body 610 inside the chamber body 410. To that end, the heating element 420 is formed by coating the outer circumferential surface of the chamber body 410 with a heat-generative paste.

To form the heating element 420, the upper ring and lower ring are first coupled to the upper and lower end, respectively, of the chamber body 410. Then, a heat-generative coating agent, e.g., carbon nanotube (CNT) paste, is applied to the outer circumferential surface of the chamber body 410. The power applied through the upper power line 130 is introduced along the upper ring 430 and generates thermal energy while passing the heat-generative coating agent-coated portion and, while flowing along the lower ring 440 and the lower power line 230, heats the chamber body 410.

To that end, the upper ring 430 and the lower ring 440 are formed of a material capable of conductance and soldering, and are configured to have jaws to allow them to be securely supported on the upper plate 110 and the lower plate 210.

The jaws formed on the outer circumferential surfaces of the upper ring 430 and the lower ring 440 allow the upper ring 430 allow for more stable contact to the upper electrode 120 and lower electrode 220 of the upper board 100 and lower board 200, so that application of power may be stably performed.

The plurality of heating chambers 400 may be configured independently from each other and are formed in a wide-top-narrow-bottom shape, and due to having small heat capacity, the heating chambers 400 may be quickly heated and cooled. Since the heating chambers 400 are separated from each other, thermal interference between adjacent heating chambers 400 may be reduced, rendering it possible to precisely and independently control temperature.

A bracket 500 seals the upper board 100 and the temperature measurement board 300 from the outside and forms air passage therebetween. To that end, the bracket 500 may be formed in a box shape to embed the temperature measurement board 300 and the upper board 100. The bracket 500 has a top opening partially formed in the top surface to expose the upper holes of the upper board 100 and a bottom opening formed in the bottom center of the lower surface.

In other words, the upper board 100, the lower board 200, the heating chambers 400, and the temperature measurement board 300 are embedded in the bracket 500. The top surface of the upper board 10 is exposed to the outside of the bracket 500, and a center portion of the temperature measurement board 300 is exposed through the bottom opening of the bracket 500 where the suction motor 520 is mounted while the rest is sealed by the bracket 500.

The bracket 500 has air inlets 510 formed along a pair of directions symmetrical with each other to allow the air to be introduced from the outside to the inside. The air inlets 510 do not contact the temperature measurement board 300 or the upper board 100. Although it is illustrated in the drawings that the air inlets 510 are formed in two opposite directions in the lower surface of the bracket 500, the air inlets 510 may be formed in two opposite side surfaces or top surface and any air inlets 510 may be formed as long as they are formed in the same size in positions symmetrical with each other with respect to the center of the bracket 500.

The suction motor 520 is coupled to the bottom opening in the bottom surface of the bracket 500 to communicate with the bottom opening.

The suction motor 520 sucks in the air from the inside of the bracket 500 and, to that end, is installed on the lower end of the bracket 500 to communicate with the bottom opening. The suction motor 520 is driven under the control of the controller, and temperature control is performed by the amount of passing air by varying the rotation speed of the suction motor 520.

If the suction motor 520 is driven, the air inside the bracket 500 is discharged to the outside through the suction motor 520 and, at this time, a negative pressure is formed inside the bracket 500 so that the external air is introduced to the inside of the bracket 500 through the air inlets 510. Preferably, the air sucked through the suction motor 520 is discharged through a separate line so as not to be mixed with the air introduced through the air inlets 510.

If the air is introduced from the outside as the suction motor 502 is driven, the introduced air flows between the heating chambers 400 between the upper board 100 and the lower board 200. The air flow cools down the heating chambers 400.

The sample plate 600 contains gene samples and is inserted to the heating chambers 400 so that heating and cooling of the gene samples may be repeated, and the sample plate 600 typically includes a plurality of tube bodies 610.

When eight tube bodies 610 are connected and used, it is called 8-strip tube, and when 12 tube bodies are connected, it is called 12-strip tube, and they are typically used.

The controller controls the power supply to the heating elements 420 of the heating chambers 400 and, to that end, the controller is configured to selectively connect the upper power lines 130 to the power source and selectively connect the lower power lines 230 to the ground so that the heating chamber 400, in which the upper ring 430 and the lower ring 440, respectively, are connected with the upper power line 130 connected with the power source and the lower power line 230 connected with the ground, generates heat.

To that end, the controller includes power source switches that are individually provided at the upper power lines 130 to connect the upper power lines 130 to the power source and ground switches that are individually provided at the lower power lines 230 to connect the lower power lines 230 to the ground.

In this case, each power source switch is assigned a serial number corresponding to the sequence number of the upper power line 130, and each ground switch is assigned a serial number corresponding to the sequence number of the lower power line 203. The temperature measurement sensor 320 has a heating address that has a composite serial number of the sequence numbers of the upper power line 130 and the lower power line 230. For example, if the upper power lines 130 have serial numbers of C1 to C12, and the lower power lines 230 have serial numbers R1 to R8, the heating addresses of the temperature measurement sensors 320 are C1R1, C1R2, C1R3,..., C12R8. The heating chambers 400 one-to-one match the temperature measurement sensors 320, that is, the heating addresses.

The controller may control the power source switches and the ground switches to be sequentially driven. For example, if the power source switches have serial numbers C1 to C12 and the ground switches have serial numbers R1 to R8, the controller first connects, first, the power source switches C1 to C2 to the power source, with the ground switch R1 remaining connected to the ground. If one cycle is ended for the power source switches, the controller then connects, sequentially, the power source switches C1 to C12 to the power source, with the ground switch R2 remaining connected to the ground. Thus, power may be sequentially supplied to the heating chambers 400 from the heating chamber 400 having the heating address C1R1 to the heating address C12R8, and this allows for a quicker response than simultaneously applying power to the heating chambers 400 and may present similar effects with a simplified configuration as compared with independently applying power to each of the heating chambers 400.

The controller receives the value measured by the temperature measurement sensor 320 and calculates the difference between the value measured by the temperature measurement sensor 320 and a target temperature value. The controller matches the pulse width of the voltage supplied to each heating chamber 400 with the heating address using the calculated temperature value and store them and, when the upper power line 130 and the lower power line 230 are connected to the power source and the ground, controls the driving time of the power source switch or ground switch to apply the voltage with the pulse width corresponding to the heating address to the upper power line 130.

More specifically, the value measured by the temperature measurement sensor 320 is equal to or lower than the target temperature value. The difference in temperature value is calculated as to how much power needs to be supplied to allow the temperature measurement value at the corresponding heating address to reach the target temperature value by a table experimentally obtained, and the calculated power value is converted into a voltage pulse width. Of course, the power value may be varied by changing the magnitude of the voltage, but it enables quicker and precise control to set voltages to the same value and then change the voltage pulse width, rather than to supply different voltages to a few tens of heating chambers 400.

In this case, as the required power value increases, i.e., the difference between the target temperature value and the value measured by the temperature measurement sensor 320 at the corresponding heating address increases, the voltage pulse width increases.

If the voltage pulse width required at the corresponding heating address is determined in the above-described manner, the controller matches the determined voltage pulse width with the heating address and stores them. When the power source switch and ground switch having the serial number of the heating address are operated, the power source switch or ground switch is connected to supply as high a voltage as the pulse width matched with the heating address and stored.

Meanwhile, the process of heating and then cooling the tube bodies 610 containing gene samples needs to be rapidly repeated several times so as to amplify the gene. To that end, the controller repeats the process of supplying the heating chamber 400 to heat the heating chamber 400 and, after stopping heating the heating chamber 400, driving the suction motor 520 to cool the heating chamber 400.

According to the present invention configured as described above, the plurality of heating chambers 400 are independently arrayed, and power is applied to the heating elements 420 formed on the outer circumferential surfaces of the heating chambers 400 to heat the tube bodies 610. Thus, heating may be quickly performed, and each heating chamber 400 may be independently heated, rendering it possible to precisely control temperature.

Further, according to the present invention, the plurality of heating chambers 400, which are arrayed, are divided into columns and rows at their upper and lower ends and may be selectively connected to the power source and the ground, so that each of the heating chambers 400 may be independently controlled for its temperature.

Further, according to the present invention, since a different temperature may be set for each heating chamber 400, it is possible to identify the reactivity for multiple temperature conditions by one test.

Further, according to the present invention, since the plurality of heating chambers 400, which are arrayed, are divided into columns and rows at their upper and lower ends and may be selectively connected to the power source and the ground, it is possible to simultaneously perform gene amplification tests with different temperature conditions.

Further, according to the present invention, since the conventional heating chambers may be replaced simply by coupling the heating chambers 400 coated with the heating elements 420 between the upper board 100 and lower board 200 which are shaped as printed circuit boards, it is possible to simplify the structure and save manufacturing costs.

Further, according to the present invention, the heating chambers 400 having a wide-top-narrow-bottom shape are directly heated, and heat is directly transferred to the tube bodies 610 tightly contacting the inside thereof. Thus, the thermal conductance time is short, and the heat capacity of the heating chambers 400 is small, so that heating and cooling may be quickly performed. Thus, it is possible to shorten the temperature cycling time and reduce gene amplification time.

### [Legend of symbols]

- 100:: upper board 110: upper plate
- 120:: upper electrode 130: upper power line
- 200:: lower board 210: lower plate
- 220:: lower electrode 230: lower power line
- 300:: temperature measurement board 310: temperature measurement plate
- 320:: temperature measurement sensor 400: heating chamber
- 410:: chamber body 420: heating element
- 430:: upper ring 440: lower ring
- 500:: bracket 510: air inlet
- 520:: suction motor 600: sample plate
- 610:: tube body

## Claims

1. A block for PCR capable of independent temperature control, comprising:
an upper board including an array of a plurality of upper through holes, upper electrodes formed at the upper holes, and a plurality of upper power lines connected to the upper electrodes;
a lower board including an array of a plurality of lower through holes, lower electrodes formed at the lower holes, and a plurality of lower power lines connected to the lower electrodes;
a temperature measurement board disposed under the lower board and including a plurality of temperature measurement sensors;
a plurality of heating chambers including chamber bodies whose lower surfaces contacting the temperature measurement sensors, heating elements formed on outer circumferential surfaces of the chamber bodies to receive electricity to generate heat, upper rings provided on the chamber bodies to be electrically conducted with the heating elements and contacting the upper electrodes, and lower rings provided on the chamber bodies to be electrically conducted with the heating chambers and contacting the lower electrodes; and
a controller selectively conducting electricity to the upper electrodes and the lower electrodes to allow the heating elements of the heating chambers to be selected heated.

2. The block for PCR of claim 1, wherein the controller includes power source switches individually provided to the upper power lines to connect the upper power lines to a power source and ground switches individually provided to the lower power lines to connect the lower power lines to a ground.

3. The block for PCR of claim 2, wherein the temperature measurement sensors have heating addresses using sequence numbers of the upper power lines and the lower power lines, as serial numbers, and wherein the controller receives measurement values from the temperature measurement sensors, matches pulse widths of voltages to be supplied to the heating chambers with the heating addresses using differences between the measurement values of the temperature measurement sensors and a target temperature value and stores the matches, and when an upper power line and a lower power line are connected to the power source and the ground, controls the power source switches and the ground switches to apply power having a pulse width corresponding to a heating address to the upper power line and the lower power line.

4. The block for PCR of claim 3, further comprising: a bracket including a top opening partially formed in an upper surface to expose the upper holes of the upper board to an outside and a bottom opening formed in a central portion of a lower surface; and a suction motor coupled to a lower end of the bottom opening of the bracket to suck air from an inside of the bracket, wherein the temperature measurement board has a plurality of through holes in a position corresponding to the bottom opening, and wherein the bracket has air inlets formed in a pair of directions symmetrical with each other not to contact the temperature measurement board or the upper board, external air introduced through the air inlets.

5. The block for PCR of claim 1, wherein the chamber bodies of the heating chambers are formed in a wide-top-narrow bottom shape.

6. The block for PCR of claim 5, wherein the upper rings and the lower rings of the heating chambers are formed of a material capable of conductance and soldering, and wherein the heating elements are formed by coating the chamber bodies with a heat-generative paste, with the upper rings and the lower rings coupled.
